# EUROPÄISCHE PATENTSCHRIFT

(11) **EP 1 949 106 B1**
(45) Veröffentlichungstag und Bekanntmachung des Hinweises auf die Patenterteilung: **25.07.2012**
(21) Anmeldenummer: 06806306.4
(22) Anmeldetag: 16.10.2006
(51) Int. Cl.: G01N 33/543, G01N 33/574, C12Q 1/68, G06F 19/00

(54) **Verfahren unter Verwendung einer Detektionseinheit, insbesondere eines Biosensors**
Method using a detection unit, in particular a biosensor
Procédé utilisant une unite de détection, notamment une biodétecteur

(30) Priorität: 29.11.2005 DE 102005057242; 22.12.2005 DE 102005062159
(43) Veröffentlichungstag der Anmeldung: 30.07.2008
(73) Patentinhaber: Farco-Pharma GmbH, 50670 Köln (DE); Universität Duisburg-Essen, 45141 Essen (DE)
(72) Erfinder: FLOHR, Hans, 56073 Koblenz (DE); RÜBBEN, Herbert, 45133 Essen (DE)
(74) Vertreter: Gesthuysen, von Rohr & Eggert
(86) Internationale Anmeldenummer: PCT/EP2006/009969
(87) Internationale Veröffentlichungsnummer: WO 2007/062719

(56) Entgegenhaltungen:
- US-A1- 2002 090 649
- NEBLING E ET AL: "Electrical detection of viral DNA using ultramicroelectrode arrays." ANALYTICAL CHEMISTRY, Bd. 76, Nr. 3, 1. Februar 2004 (2004-02-01), Seiten 689-696, XP002414580
- LIN J ET AL: "Electrochemical and chemiluminescent immunosensors for tumor markers." BIOSENSORS & BIOELECTRONICS, Bd. 20, Nr. 8, 15. Februar 2005 (2005-02-15), Seiten 1461-1470, XP002414581
- WILSON M S: "Electrochemical immunosensors for the simultaneous detection of two tumor markers." ANALYTICAL CHEMISTRY, Bd. 77, Nr. 5, 1. März 2005 (2005-03-01), Seiten 1496-1502, XP002414582
- ZHENG G ET AL: "Multiplexed electrical detection of cancer markers with nanowire sensor arrays." NATURE BIOTECHNOLOGY, Bd. 23, Nr. 10, Oktober 2005 (2005-10), Seiten 1294-1301, XP002414583
- KOJIMA K ET AL: "ELECTROCHEMICAL PROTEIN CHIP WITH ARRAYED IMMUNOSENSORS WITH ANTIBODIES IMMOBILIZED IN A PLASMA-POLYMERIZED FILMS" ANALYTICAL CHEMISTRY, Bd. 75, Nr. 5, 1. März 2003 (2003-03-01), Seiten 1116-1122, XP001170271
- RHODES D R ET AL: "Multiplex biomarker approach for determining risk of prostate-specific antigen-defined recurrence of prostate cancer" JOURNAL OF THE NATIONAL CANCER INSTITUTE, Bd. 95, Nr. 9, 7. Mai 2003 (2003-05-07), Seiten 661-668, XP002414584
- MILLER J C ET AL: "The application of protein microarrays to serum diagnostics: Prostate cancer as a test case" DISEASE MARKERS, Bd. 17, Nr. 4, 2001, Seiten 225-234, XP009020168
- GUO Q M: "DNA MICROARRAY AND CANCER" CURRENT OPINION IN ONCOLOGY, Bd. 15, Nr. 1, Januar 2003 (2003-01), Seiten 36-43, XP008045590
- FU L M ET AL: "Multi-class cancer subtype classification based on gene expression signatures with reliability analysis" FEBS LETTERS, Bd. 561, Nr. 1-3, 12. März 2004 (2004-03-12), Seiten 186-190, XP004495476
- GRIMM M-O ET AL: "[Microarrays]" DER UROLOGE, Bd. 43, Nr. 6, Juni 2004 (2004-06), Seiten 653-658, XP002414586
- DUDDA-SUBRAMANYA R ET AL: "Clinical applications of DNA microarray analysis." JOURNAL OF EXPERIMENTAL THERAPEUTICS & ONCOLOGY, Bd. 3, Nr. 6, November 2003 (2003-11), Seiten 297-304, XP002414587
- Rainer Hintsche: "Bioanalytik vor Ort mit elektrischen Biochips", HIGHCHEM hautnah - Aktuelles aus der Analytischen Chemie, 4 October 2005 (2005-10-04), pages 1-2, XP55004061, Retrieved from the Internet: URL:http://www.aktuelle-wochenschau.de/200 5/woche40/woche40.html [retrieved on 2011-08-03]
- Anonymous: "Das Labor für unterwegs", Life Science Nord 1/2005, 1 March 2005 (2005-03-01), pages 9-11, XP55004068, Retrieved from the Internet: URL:http://www.life-science-nord.net/filea dmin/lsn/pdf/Magazin/LifeScienceNord_1-05. pdf [retrieved on 2011-08-03]

## Beschreibung

Die vorliegende Erfindung betrifft ein Verfahren zur Ermittlung des Risikos, an einer Krankheit zu erkranken, bzw. zur Prognose eines Krankheitsverlaufs sowie zur Prognose von individuellen Arzneimittelwirkungen sowie ein Verfahren zur Erstellung einer Referenzdatenbank für Krankheiten, welche eine Vielzahl krankheitsspezifischer Profile von einer Vielzahl von Patienten umfaßt.

Eine Erkrankung geht häufig mit der Veränderung physiologischer Parameter einher: So liegt beispielsweise bei entzündlichen Krankheiten oftmals eine signifikante Veränderung des Blutbildes, beispielsweise eine deutliche Erhöhung der Anzahl weißer Blutkörperchen sowie ein Auftreten bzw. eine Freisetzung bestimmter Entzündungsmediatoren und/oder Antigene und/oder Antikörper, vor. Eine gezielte qualitative und quantitative Analyse derartiger Veränderungen kann - beispielsweise in Verbindung mit weiteren diagnostischen Maßnahmen - zu einer spezifischen Bestimmung bzw. Diagnose der vorliegenden Erkrankung herangezogen werden, so daß auf dieser Basis eine gezielte kurative und/oder therapeutische Behandlung unter Auswahl spezifischer Arzneimittel eingeleitet werden kann.

Neben entzündlichen Erkrankungen, welche beispielsweise auf virale oder bakterielle Infektionen zurückgehen, sind insbesondere Tumor- und Krebserkrankungen mit der Freisetzung spezifischer Moleküle bzw. Marker verbunden, wobei es sich hierbei insbesondere um spezifische Proteine handelt, welche in bezug auf die Tumorentstehung und -entwicklung sowie dessen Metastasierung eine Rolle spielen und für das Auftreten von Tumorerkrankungen charakteristisch sind. In diesem Zusammenhang können beispielhaft sogenannte, insbesondere durch Tumorzellen im hohen Maße synthetisierte Wachstumsfaktoren genannt werden, wie beispielsweise VEGF ("*Vasular Endothelial Growth Factor*"), dem eine entscheidende Rolle zur Bildung neuer Blutgefäße zur Versorgung eines Tumors zukommt. Weitere, durch den Tumor gebildete bzw. tumorinduzierte Proteine - synonym auch als Marker, Markerproteine bzw. Markersubstanzen bezeichnet - sind beispielsweise spezifische Zellzyklusregulatoren, Chemokinrezeptoren sowie Signaltransduktionsfaktoren sowie eine große Breite weiterer Verbindungen, welche insgesamt ein sogenanntes tumor- bzw. krebsspezifisches Markerprofil ergeben, anhand dessen eine Tumorerkrankung identifiziert bzw. im Hinblick auf die weitere Tumorenrivicklung charakterisiert werden kann. Zu diesen Markersubstanzen zählt auch das sogenannte *Prostataspezifische Antigen* (PSA), welches einen spezifischen Indikator für ein Prostatakarzinom darstellt und anhand dessen Bestimmung beispielsweise eine Vorhersage in bezug auf die Tumor- bzw. Krebsentwicklung, beispielsweise in bezug auf das Risiko einer Lymphknotenmetastasierung, getroffen werden kann.

Im allgemeinen kann somit aufgrund der Spezifität eines Markerprofils dieses beispielsweise zur Identifizierung und Charakterisierung von Tumor- bzw. Krebserkrankungen herangezogen werden.

Im Stand der Technik sind zur Ermittlung von Markern bzw. Markerprofilen zahlreiche biochemische Identifizierungs- bzw. Detektionsverfahren beschrieben, wie das sogenannte Festphasenimmunoassay-Verfahren sowie das ELISA-Verfahren ("*Enzyme-Linked Immunosorbent Assay*")*,* bei welchen die Detektion spezifischer Proteine bzw. Antigene immunologisch und letztlich über radioaktive Markierungen oder Fluoreszenzmarkierung erfolgt. Eine weitere immunbiologische Technik stellt das sogenannte *Western-Blotting* dar.

Derartige Nachweissysteme weisen jedoch den gravierenden Nachteil auf, daß sie verfahrenstechnisch aufwendig sind, da beispielsweise eine arbeitsintensive Präparation bzw. Aufarbeitung der zu analysierenden Probe notwendig ist. Darüber hinaus sind die vorgenannten Verfahren des Standes der Technik zeit- und kostenintensiv und führen oftmals nur zu einer quantitativen Bestimmung der zu detektierenden Substanz. Auch sind diese Verfahren nicht oder nicht ohne weiteres automatisierbar.

Zudem ist im Stand der Technik beispielsweise die Bestimmung spezifischer hamblasenkarzinornassoziierter Antigene durch die Entwicklung monoklonaler Antikörper möglich, welche aus der Fusion antikörperproduzierender Zellen mit immortalisierten Zellen in vitro produziert werden können. Dabei werden die von sogenannten Hybridomzellen produzierten Antikörper mit einem zweiten Antikörper gekoppelt, um die sogenannte Antigen/Antikörper-Reaktion an der Zielzelle optisch sichtbar oder zytometrisch-photometrisch meßbar zu machen.

Eine weitere biochemische, insbesondere molekulazgenetische Technik zur Detektion bestimmter DNA-Stränge bzw. -Abschnitte ist die sogenannte Fluoreszenz-in-situ-Hybridisierung (FISH) sowie die hieraus hervorgehende Weiterentwicklung, die sogenannte vergleichende genomische Hybridisierung (CGH). Derartige Verfahren sind jedoch auf genetisches Material, wie DNA, beschränkt. Die Detektion erfolgt gleichermaßen photometrisch. Zudem ist eine zeitnahe, beispielsweise intraoperativ durchführbare Markeranalyse mit diesen Techniken nicht realisierbar. Eine zeitnahe Analyse kann jedoch im Rahmen einer individuellen Prognoseabschätzung bereits zu einer intraoperativen Therapieoptimierung führen.

Eine weitere Entwicklung in bezug auf die spezifische Bestimmung von Proteinen bzw. Marker stellen sogenannte "*Lab-on-a-chip*"-Lösungen dar, bei denen auf einem Träger ein entsprechender Proteinnachweis erfolgen kann. Jedoch weisen derartige Detektionsvorrichtungen keine integrierte Sensorik auf, und die Auslesung erfolgt nach Pzobenauftrag deutlich zeitversetzt bzw. "offline" (extern) mittels optischer Detektion unter Verwendung externer und aufwendiger optischer Lese- und Auswertevorrichtungen, ohne daß diese Vorrichtungen auf der Detektionsvorrichtung integriert sind.

Demnach sind derartige Systeme apparativ aufwendig, und eine zeitnahe Ermittlung eines Markerprofils ist mit derartigen Systemen ebenfalls nur begrenzt möglich.

Insgesamt ist somit im Stand der Technik bisher kein System bekannt, welches portabel ist und mit welchem automatisch und zumindest semikontinuierlich eine biosensorische Gewebstypisierung, insbesondere zur Ermittlung eines Markerprofil, durchgeführt werden kann.

Weiterhin betrifft die Publikation gemäß Rhodes D. R. et al., 2003, J. Nat. Canc. Inst., 95(9), 661-668 einen Multiplexbiomarkeransatz zur Risikobestimmung des Wiederauftretens von Prostatakrebs auf Basis spezieller prostataspezifischer Antigene. Diesbezüglich werden *Tissue Micro Arrays* (TMAs) eingesetzt. Die angeführten TMAs beruhen im allgemeinen auf immunhistochemische bzw. fluoreszenzspezifische Verfahren und Verfahren der in situ-Hybridisierung. Dabei wird insbesondere auf immunhistochemische Verfahren abgestellt.

Die Publikation gemäß Miller J. C. et al., 2001, Disease Markers, 17 (4), 225-234 stellt auf farbgebende Verfahren zur Bestimmung von Proteinen ab (vgl. Abschnitt 5: "*5*. *Protein microarrays for highly multiplexer protein detection*", insbesondere "... *were labled by covalent attachment of spectrallyresolvable fluorescent dyes.*"),

Zudem betrifft die US 2002/0090649 A1 eine Vorrichtung zur elektrischen Detektion bzw. Erfassung von molekularen Interaktionen zwischen immobilisierten Probenmolekülen (*probe molecules*) einerseits und Zielmolekülen (*target molecules*) in einer Testlösung andererseits. Die Vorrichtung soll dabei ein Trägersubstrat mit einer Anordnung von Testfeldern und damit in Verbindung stehenden Eingangselektroden sowie mit den Eingangselektroden in elektrochemischer Verbindung stehenden Ausgangselektroden sowie Verbindungseinheiten (*linker moieties*), welche mit den Eingangs- bzw. Ausgangselektroden im Bereich der Testfelder in Verbindung stehen, umfassen. Gemäß dieser Entgegenhaltung sollen die mit den zu erfassenden Molekülen interagierenden Probenmoleküle auf der Verbindungseinheit immobilisiert sein, wobei eine molekulare Interaktion zwischen den immobilisierten Probenmolekülen und den zu erfassenden Molekülen in Form einer elektrischen Signaldifferenz erfasst werden soll.

Vor diesem technischen Hintergrund besteht nunmehr die Aufgabe der vorliegenden Erfindung, ein Verfahren zur Erstellung einer Referenzdatenbank für Krankheiten aus der Gruppe von Tumorerkrankungen des Urogenitaltrakts mit einer Vielzahl von krankheitsspezifischen Profilen einer Vielzahl von Patienten sowie ein Verfahren zur Ermittlung eines Risikos, an solchen Krankheiten zu erkranken, bzw. zur Prognose eines Krankheitsverlaufs bzw. zur Prognose von individuellen Arzneimittelwirkungen bei solchen Krankheiten unter Verwendung einer Detektionseinheit, insbesondere eines Biosensors, bereitzustellen, wobei die zuvor geschilderten Nachteile des Standes der Technik zumindest teilweise vermieden oder aber zumindest abgeschwächt werden sollen. Insbesondere sollen solche Verfahren der vorgenannten Art bereitgestellt werden, mit welchen spezifische Ziel- bzw. Markermoleküle in qualitativer und/oder quantitativer Weise erfaßt werden können, so daß auf dieser Basis ein Markerprofil einer biologischen Probe, beispielsweise eines Tumorgewebes oder einer Körperflüssigkeit (z. B. Blut oder Urin), erstellt werden kann. Dabei soll die dabei erfindungsgemäß eingesetzte Detektionseinheit vorteilhafterweise sowohl spezifische Erfassungskomponenten als auch zur Detektion erforderliche Sensorkomponenten umfassen, wobei die Detektion insbesondere unter Ausgabe eines elektrischen Meßsignals erfolgen soll. Insbesondere soll sich die erfindungsgemäß eingesetzte Detektionseinheit zur Detektion von Zielmolekülen eignen, welche für das Auftreten bzw. Vorliegen von Tumor- bzw. Krebserkrankungen oder aber von entzündlichen Erkrankungen des Urogenitaltraktes charakteristisch sind.

Zur Lösung der zuvor geschilderten Aufgabenstellung schlägt die vorliegende Erfindung ein Verfahren zur Erstellung einer Referenzdatenbank für Tumor- bzw. Krebserkrankungen und entzündlichen Erkrankungen des Urogenitaltraktes gemäß Anspruch 1 vor. Weitere, vorteilhafte Ausgestaltungen sind Gegenstand der jeweiligen Unteransprüche.

Weiterhin ist Gegenstand der vorliegenden Erfindung ein Verfahren zur Ermittlung des Risikos, an einer Krankheit zu erkranken, bzw. zur Prognose eines Krankheitsverlaufs bzw. zur Prognose von individuellen Arzneimittelwirkungen gemäß Anspruch 4.

Im Rahmen der erfindungsgemäßen Verfahren kommt eine Detektionseinheit zum Einsatz, welche insbesondere als Biosensor ausgebildet ist und mit spezifischen Fängermolekülen ausgestattet ist. Die Fängermoleküle sind derart ausgestaltet, daß sie mit Ziel- bzw. Markermolekülen in spezifischer Art und Weise interagieren können.

Eine Besonderheit der erfindungsgemäß verwendeten Detektionseinheit ist darin zu sehen, daß ein insbesondere elektrisches Meßsignal infolge der Wechselwirkung zwischen dem Fängermolekül einerseits und dem Ziel- bzw. Markermolekül andererseits erzeugt wird. Zu diesem Zweck weist die erfindungsgemäß eingesetzte Detektionseinheit einen Träger auf, wobei auf dem Träger eine Meßeinrichtung, welche zur Erfassung der Wechselwirkung zwischen Fängermolekül einerseits und Ziel- bzw. Markermolekül andererseits sowie zur Erzeugung eines insbesondere elektrischen Meßsignals infolge der Wechselwirkung dient, angeordnet ist. Die Detektionseinheit zeichnet sich weiterhin dadurch aus, daß auf dem Träger bzw. auf der Meßeinrichtung Fängermoleküle immobilisiert sind. Die Fängermoleküle sind auf der Meßeinrichtung angeordnet. Die Zielmoleküle sind für das Auftreten bzw. Vorliegen von Tumorerkrankungen, insbesondere Krebserkrankungen, des Urogenitaltraktes, charakteristisch.

Mit Hilfe der erfindungsgemäß eingesetzten Detektionseinheit ist es möglich, eine rasche Identifizierung von gewebstypischen Markern zu realisieren, was weiterführende Aussagen in bezug auf die zuvor genannten Erkrankungen, beispielsweise bereits vor oder während einer Tumoroperation, ermöglicht. Darüber hinaus zeichnet sich die Detektionseinheit dadurch aus, daß sie in ein portables Ansteuerungs- und Auswertesystem integriert werden kann und darüber hinaus eine automatisierte bzw. selbstständige Messung mehrerer paralleler Proben möglich ist.

Eine zentrale Idee der vorliegenden Erfindung ist darin zu sehen, daß eine Detektionseinheit eingesetzt wird, mit welcher in einer Messung eine Vielzahl spezifischer Ziel- bzw. Markermoleküle ermittelt werden kann, so daß es in einfacher Weise möglich ist, ein komplexes Markerprofil einer Probe, beispielsweise einer Gewebeprobe eines Tumors, zu erstellen. Gleichermaßen ist es aber auch möglich, daß mehrere verschiedene Proben - beispielsweise Gewebeproben von Tumoren verschiedener Patienten - auf einer einzigen Detektionseinheit untersucht werden können, wobei diese dann - wie nachfolgend geschildert - vorzugsweise kompartimentiert ist. Dabei vereint die Detektionseinheit gewissermaßen in einer Vorrichtung sowohl erkennungsspezifische als auch sensorspezifische Komponenten. Infolge der Erkennung von Zielmolekülen werden Sensorsignale vorzugsweise in elektrischer Form ausgegeben, so daß die Wechselwirkung zwischen Fängermolekülen und Zielmolekülen quantitativ und qualitativ analysiert werden kann und auf dieser Basis Rückschlüsse auf die Zielmoleküle bzw. die Marker möglich sind.

Die Detektionseinheit ist somit speziell auf die Erfassung solcher Zielmoleküle, welche für das Auftreten bzw. Vorliegen von Tumor- bzw. Krebserkrankungen des Urogenitaltraktes charakteristisch sind, ausgelegt bzw. sozusagen maßgeschneidert. Hierin ist eine maßgebliche, zentrale Idee der vorliegenden Erfindung zu sehen.

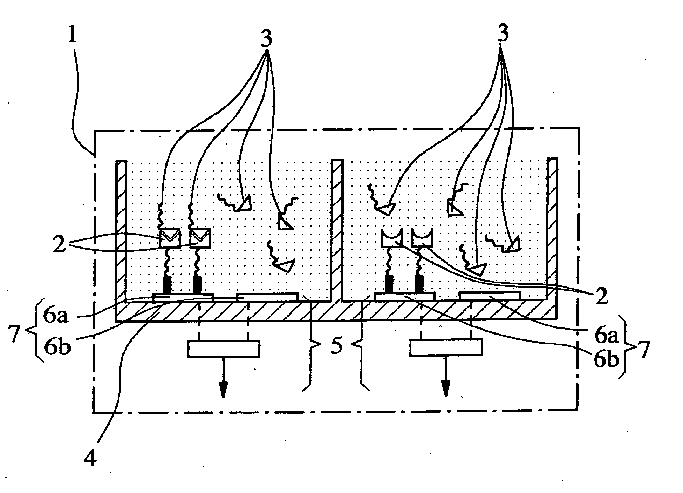

Weitere Vorteile, Merkmale, Eigenschaften und Aspekte der vorliegenden Erfindung ergeben sich aus der nachfolgenden Beschreibung von bevorzugten Ausführungsformen auf Basis der angefügten Figur. Die einzige Figur zeigt einen schematischen Querschnitt der erfindungsgemäß eingesetzten Detektionseinheit 1 gemäß einer erfindungsgemäß bevorzugten Ausführungsform, welche im vorliegenden Fall mit einer Probe beaufschlagt ist und Ziel- bzw. Markermoleküle 3 enthält.

In der einzigen Figur werden für gleiche oder ähnliche Teile die gleichen oder dieselben Bezugszeichen verwendet, wobei entsprechende Eigenschaften und Vorteile erreicht werden, auch wenn eine wiederholte Beschreibung aus Vereinfachungsgründen weggelassen ist.

Die Figur zeigt eine erfindungsgemäß verwendbare Detektionseinheit 1, insbesondere einen Biosensor, bei welchem ein insbesondere elektrisches Meßsignal infolge einer Wechselwirkung zwischen Fängermolekülen 2 einerseits und Zielmolekülen 3 andererseits erzeugt wird. Die Figur zeigt, daß die Detektionseinheit 1 einen Träger 4 aufweist, wobei auf dem Träger 4 eine Meßeinrichtung 5 angeordnet ist. Die Meßeinrichtung 5 dient der Erfassung der Wechselwirkung zwischen Fängermolekülen 2 einerseits und Zielmolekülen 3 andererseits sowie zur Erzeugung eines infolge dieser Wechselwirkung generierten, insbesondere elektrischen Meßsignals. Die Fängermoleküle 2 sind - wie der Figur weiterhin zu entnehmen ist - auf dem Träger 4 bzw. auf der Meßeinrichtung 5, vorzugsweise auf der Meßeinrichtung 5, immobilisiert. Dabei sind die Fängermoleküle 2 geeignet, in spezifischer Weise mit Zielmolekülen 3, welche für das Auftreten bzw. Vorliegen von Krankheiten aus der Gruppe von Tumorerkrankungen, insbesondere Krebserkrankungen, des Urogenitaltraktes charakteristisch sind, in Wechselwirkung zu treten. Die Detektionseinheit 1 ist hierdurch speziell auf die Erfassung solcher Zielmoleküle, welche für das Auftreten bzw. Vorliegen von Tumor- bzw. Krebserkrankungen des Urogenitaltraktes charakteristisch sind, ausgerichtet.

Was die erfindungsgemäß eingesetzte Detektionseinheit 1 als solche anbelangt, so wird diese synonym auch als Biochip, Biosensor bzw. elektrochemischer Sensor bezeichnet, insbesondere da es sich bei der Detektionseinheit 1 um einen hochsensitiven und selektiven Sensor beispielsweise für Affinitätsbindungen von auf dem Sensor immobilisierten bzw. fixierten Molekülen (Fängermolekülen 2) einerseits und in einer Probe befindlichen Ziel- bzw. Markermolekülen 3 andererseits handelt, wobei infolge der zuvor beschriebenen Interaktion ein insbesondere elektrisches Meßsignal ausgegeben wird.

Im Rahmen der vorliegenden Erfindung bezeichnet der Begriff "Biosensor" somit insbesondere eine Kopplung biologischer Komponenten (z. B. Fängermoleküle 2) zur spezifischen Erkennung eines Analyten (z, B. Zielmoleküle 3) mit einem insbesondere physikalischen Transduktor bzw. Signalwandler. Dabei vereinigt der Biosensor in sich die hohe Spezifität biologischer Systeme mit großer Nachweisempfindlichkeit physikalischer Systeme. Der Biosensor wandelt die biochemische Information eines Substrates (z. B. Zielmoleküle 3) in ein physikalisch quantifizierbares Signal um, vorzugsweise in ein elektrisches Signal, das einer elektronischen Verstärkung zugänglich ist. Für weitere Einzelheiten in bezug auf Biosensoren kann verwiesen werden auf Römpp Lexikon Biotechnolgie und Gentechnik. 2, Auflage, 1999, Georg Thieme Verlag Stuttgart/New York, Seite 120, Stichwort: "Biosensoren", Der vorstehende Begriff "Analyt" wird erfindungsgemäß als synonyme Bezeichnung für das Zielmolekül 3 verwendet.

Die erfindungsgemäß eingesetzte Detektionseinheit 1 - wie nachfolgend noch ausführlich erörtert - weist Meßelektroden 6a, 6b auf, welche als ein Bestandteil der Meßeinrichtung 5 auf den Träger 4 aufgebracht sind und in Kontakt mit einer zu analysierenden Probe stehen. Dabei sind die Fängermoleküle 2 auf den Meßelektroden 6a, 6b immobilisiert.

Was die erfindungsgemäß verwendeten Fängermoleküle 2 anbelangt, so handelt es sich hierbei gewissermaßen um eine Biokomponente (synonym auch als Biomolekül bezeichnet), welche imstande ist, mit den Ziel- bzw. Markermolekülen 3 zu interagieren bzw. hiermit in Wechselwirkung zu treten. Dabei kann der Begriff "Fängermolekül" einerseits eine Vielzahl von identischen Fängermolekülen sowie andererseits ein Ensemble unterschiedlicher Spezies von Biokomponenten umfassen. Der Begriff "Fängermolekül" betrifft somit mit anderen Worten sowohl eine Sorte von Biokomponenten (z. B. einen speziellen Antikörper) als auch eine Mischung verschiedener Biokomponenten (z. B. eine Mischung verschiedener Antikörper). Demnach liegt es im Rahmen der vorliegenden Erfindung, wenn der Träger 4 bzw. die Meßeinrichtung 5 und besonders bevorzugt die Meßelektroden 6a, 6b - je nach Anwendungsfall - mit einer Vielzahl identischer oder mit einer Mischung unterschiedlicher Fängermolekülen beaufschlagt ist bzw. sind. Da die erfindungsgemäße Detektionseinheit 1 nicht auf eine einzige Meßeinrichtung 5 beschränkt ist, sondern gleichermaßen eine Vielzahl von vorzugsweise unabhängigen Meßeinrichtungen 5 aufweisen kann, ist es möglich, daß die erfindungsgemäße Detektionseinheit 1 verschiedene Meßeinrichtungen 5 mit jeweils voneinander verschiedenen Fängermolekülen 2 je Meßeinrichtung 5 aufweist.

Die erfindungsgemäß eingesetzte Detektionseinheit 1 eignet sich aufgrund ihrer speziellen Konzeption für eine vorzugsweise elektrische Multianalytmessung, wobei die Detektionseinheit 1 zu diesem Zweck mehrere unterschiedliche Meßeinrichtungen 5 umfaßt, auf welchen jeweils unterschiedliche Fängermoleküle 2 gemäß der vorgenannten Begriffsdefinition immobilisiert sind. Sofern eine Untersuchung verschiedener Proben, beispielsweise von verschiedenen Patienten bzw. von verschiedenen Geweben, vorgesehen ist, werden die einzelnen Meßeinrichtungen 5 durch Trennwände bzw. Kompartimentierungen voneinander getrennt, so daß eine kompartimentierte Detektionseinheit 1 resultiert. Die Kompartimente weisen dann vorzugsweise Volumina im Bereich von wenigen Nanolitern auf. Da die Detektionseinheit 1 mehrere Meßeinrichtungen 5 aufweist, sind diese einzeln bzw. getrennt voneinander steuerbar, d. h. die jeweiligen Meßeinrichtungen 5 sind getrennt voneinander z. B. mit elektrischen Potentialen beaufschlagbar bzw. Meßsignale sind unabhängig voneinander aus den jeweiligen Meßeinrichtungen 5, beispielsweise über Meßelektroden 6a, 6b, auslesbar.

Die erfindungsgemäß vorgesehene Meßeinrichtung 5 umfaßt somit gemäß einer erfindungsgemäß bevorzugten Ausführungsform elektrische Meßelektroden (6a, 6b) sowie einen Teil des Trägers 4 und kann gegebenenfalls beispielsweise durch Wandungen der Trägerstruktur ein abgetrenntes Areal der erfindungsgemäßen Detektionseinheit 1 ausbilden. Wie nachfolgend noch beschrieben, kann die Meßeinrichtung 5 als ein sogenanntes Elektrodenarray ausgebildet sein.

Was die Fängermoleküle 2 als solche betrifft, so handelt es sich hierbei um Antikörper. Die Auswahl der Fängermoleküle 2 ist erfindungsgemäß in Abhängigkeit von den zu detektierenden Zielmoleküle 3 durchzuführen. Dabei sind die Fängermoleküle 2 derart ausgebildet, daß sie mit den Zielmolekülen 3 in Wechselwirkung treten können, so daß eine spezifische Anlagerung bzw. Bindung der Zielmoleküle 3 an die Fängermoleküle 2 erfolgen kann. Diesbezüglich sollten die Fängermoleküle 2 derart ausgebildet sein, daß sie durch ihre hohe Spezifität ausschließlich mit den entsprechenden Zielmolekülen 3 interagieren, sofern diese in der Probe vorhanden sind.

Die Bindung zwischen Fängermolekülen 2 und Zielmolekülen 3 kann physikalisch und/oder chemisch, beispielsweise durch elektrostatische und/oder interionische Wechselwirkung oder durch Ausbildung kovalenter Bindungen erfolgen, so daß gewissermaßen ein Fängermolekül/Zielmolekül-Komplex ("Schlüssel/Schloß-Prinzip") resultiert, was zu einem insbesondere elektrochemischen Detektionsprozeß, beispielsweise zur Ausgabe eines elektronischen Meßsignals, führt. Mit anderen Worten sind die Fängermoleküle 2 sozusagen komplementär zu den Zielstrukturen, d. h. komplementär zu den Zielmolekülen 3, auszuwählen. Fängermoleküle 2 und Zielmoleküle 3 stellen somit gewissermaßen aufeinander abgestimmte, interaktionsfahige Biokomponenten dar. Sofern die Zielmoleküle 3 beispielsweise Proteine mit Antigenfunktion sind, sollten die Fängermoleküle 2 entsprechend für diese Antigenstruktur spezifische Antikörper sein.

Die Fängermoleküle 2 sind derart ausgelegt, daß sie geeignet sind, mit den Zielmolekülen 3 in Wechselwirkung zu treten, welche für das Auftreten bzw. Vorliegen einer Tumorerkrankung, insbesondere einer Krebserkrankung, des Urogenitaltraktes charakteristisch sind. In diesern Zusammenhang handelt es sich bei der Tumorerkrankung, insbesondere Krebserkrankung, beispielsweise und in nichtbeschränkender Weise um ein Urethelkarzinom, Prostatakarzinom bzw. Nierenkarzinom. Was die Tumor- bzw. Krebserkrankung betrifft, so umfaßt diese Primärtumore, Metastasen, Präkanzerosen (Krebsvorstufen), benigne und maligne Tumore und dergleichen. Somit sind die Fängermoleküle 2 spezifische Antikörper und die Zielmoleküle 3 Antigene. Die Zielmoleküle 3 sind vorzugsweise für die jeweilige Tumor- bzw. Krebserkrankung spezifische Proteinstrukturen bzw. sogenannte Markerproteine, d. h. aufgrund oder im Zusammenhang mit der spezifischen Tumor- bzw. Krebserkrankung auftretende Proteine, welche beispielsweise durch den Tumor selbst synthetisiert und/oder freigesetzt werden oder aber beispielsweise durch tumorfrernde Gewebe bzw. Organe gewissermaßen als Reaktion auf die Tumorentstehung bzw. -entwicklung synthetisiert und/oder freigesetzt werden. So kann das Zielmolekül 3 beispielsweise das zuvor genannte *Prostataspezifische Antigen* (PSA) sein, welches ein spezifisches Markerprotein für ein Prostatakarzinom darstellt. Darüber hinaus kann es sich bei dem Zielmolekül 3 beispielsweise und in nichtbeschränkender Weise auch um ein E-Cadherin oder Catenin, wie B-Catenin, handeln. Darüber hinaus kann das Zielmolekül 3 ein Wachstumsfaktor, wie HER-2/neu, oder aber ein Proto-Onkogen, wie Cyclin-D, sein. Darüber hinaus kommen für das Zielmolekül 3 spezifische Wachsturusfaktoren, wie EGF-R, sowie Tumorsupressorgene bzw. -genprodukte, wie p53 und PTEN, sowie Zellzyklusregulatoren, wie p27Kip und Ki67, in Betracht. Weiterhin kann das Zielmolekül 3 ein Chemokinrezeptor, wie CXCR4 oder CCR7, oder ein Signaltransduktionsfäktor, wie Rho-Kinase, Rho A, Rho B, Rho C, Epac 1, Epac 2, H-Ras, Raf-Kinase, Rap 1 bzw. Rap 2, sein. Auch Motilitätsfaktoren, wie Zellmotilitätsfaktoren, beispielsweise Pecam bzw. Vimentin, und Angiogenesefaktoren, wie UPA (PLAU), kommen als Zielmolekül 3 in Betracht.

In diesem Zusammenhang wird den vorgenannten Ziel- bzw. Markermolekülen 3 eine hohe Relevanz in bezug auf eine Tumor- bzw. Krebserkrankung zugesprochen, So stellt die von den Zellen eines Primärtumors gebildete Molekülgruppe der zuvor beschriebenen vaskulären endothelialen Wachstumsfaktoren (VEGF) Schlüsselmoleküle in bezug auf eine lymphogene Tumorzellausbreitung dar. Zudem gibt es Hinweise, daß die Familie der zuvor genannten Chemokinrezeptoren eine zentrale Rolle in bezug auf eine lymphogene Metastasierung, wie Migration, Invasion und Proliferation, spielen. In Kenntnis dieser spezifischen Marker ist somit ein gezielter Rückschluß auf die jeweilige Tumorart bzw. eine spezifische Charakterisierung des Tumors möglich, so daß vor diesem Hintergrund spezifische therapeutische Ansätze der Tumorbehandlung abgestimmt bzw. eingesetzt werden können, wie beispielsweise eine gezielte Hemmung der lymphogenen Tumorzellausbreitung durch selektive Inhibition von Wachstumsfaktor- und/oder Chemokinrezeptoren. Insgesamt kann somit mittels der Detektionseinheit 1 ein spezifisches Markerprofil bzw. ein spezifisches Antigenprofil einer Probe, insbesondere eines Tumors und dergleichen, erstellt werden, so daß gewissermaßen eine biosensorische Krebsschnellerkennung bzw. eine Schnellidentifizierung von Tumor- bzw. Tumorgewebseigenschaften möglich ist, auf deren Basis beispielsweise gezielte therapeutische Schritte eingeleitet werden können.

Wie zuvor angesprochen, sind die Fängermoleküle 2 auf dem Träger 4 und/oder der Meßeinrichtung 5, insbesondere auf der Meßeinrichtung 5, immobilisiert. Im Rahmen der vorliegenden Erfindung sind die Fängermoleküle 2 auf den Meßelektroden 6a, 6b der Meßeinrichtung 5 immobilisiert, wobei es gleichermaßen möglich ist, daß die Fängermoleküle 2 auch auf den Teilen des Trägers 4 immobilisiert sind, welche die Meßeinrichtung 5 ausbilden. Hierdurch ist eine hohe Fängermolekülbeladung realisierbar, was die Sensoreigenschaften zusätzlich verbessert.

Gemäß einer erfindungsgemäß bevorzugten Ausführungsform erfolgt die Immobilisierung der Fängermoleküle 2 auf die zuvor genannten Strukturen phsikalisch und/oder chemisch, wobei eine chemische Fixierung erfindungsgemäß bevorzugt ist. Die Immobilisierung kann in diesem Zusammenhang beispielsweise mittels Selbstanordnung (*self-assembling*), Elektropolymerisation oder über Grold/Thiol-Bindungen erfolgen. Weitere Fixierungsmöglichkeiten der Fängermoleküle 2 können Adhäsion, Adsorption sowie spezifische Kondensationsreaktionen, wie beispielsweise Silanisierung in bezug auf den Träger 4, sein. Mit anderen Worten wird eine Modifizierung bzw. Belegung der Oberflächen des Trägers 4 bzw. der Meßeinrichtung 5 bzw. der Meßelektroden 6a, 6b durch kovalente Bindung oder Adhäsion an die metallischen oder nichtmetallischen Oberflächen oder an die Wandungen der entsprechenden Komponenten erreicht, wobei die Fängermoleküle 2 z. B. als Monolayer oder Multilayer aufgebracht ist.

Wie die Figur weiterhin zeigt, weist die Detektionseinheit 1 mindestens zwei Meßelektroden 6a, 6b auf. Das Material der Meßelektroden 6a, 6b ist ein Edelmetall, wobei Gold, Platin bzw. Iridium erfindungsgemäß bevorzugt sind. Die Meßeinrichtung 5 weist mindestens zwei Meßelektroden 6a, 6b auf, die vorzugsweise ein Elektrodenpaar 7 (synonym auch als Paar von Meßelektroden bezeichnet), beispielsweise eine Anode und eine Kathode, bilden. Weiterhin kann es erfindungsgemäß vorgesehen sein, daß die Meßeinrichtung 5 eine Vielzahl Meßelektroden 6a, 6b bzw. Elektrodenpaare 7 aufweist. Die Meßelektroden 6a, 6b können auf den Träger 4 aufgebracht bzw. teilweise in den Träger 4 eingelassen sein, wobei es erfindungsgemäß gewährleistet sein sollte, daß zumindest ein Teil der Meßelektroden 6a, 6b an der Oberfläche des Trägers 4 zu liegen kommt.

Um die Meßelektroden 6a, 6b beispielsweise mit einem elektrischen Potential zu beaufschlagen bzw. um elektrische Meßsignale ableiten zu können, weisen die Meßelektroden 6a, 6b vorzugsweise Anschlußleitungen auf, welche gleichermaßen auf den Träger 4 aufgebracht oder in diesen eingelassen sein können. Dabei können die Anschlußleitungen beispielsweise an den Randbereichen der Detektionseinheit 1 bzw. des Trägers 4 flächenförmig vergrößert sein, um beispielsweise mit weiteren elektrischen Geräten, z. B. über Steckverbindungen, verbunden zu werden.

Zudem ist es im Rahmen der vorliegenden Erfindung möglich, daß die Detektionseinheit 1 bzw. die Meßeinrichtung 5 über weitere Arbeits-, Gegen- und/oder Referenzelektroden verfügt, wobei die Meßelektroden 6a, 6b beispielsweise gegenüber diesen Elektroden polarisiert werden können. Beispielsweise kann eine Silber/Silberchlorid-Elektrode als Referenzelektrode eingesetzt werden. Durch die Ergänzung der Meßelektroden 6a, 6b um weitere Hilfselektroden können beispielsweise gleichzeitig sowohl Elemente zur Ausführung elektrophoretischer Transportvorgänge der zu analysierenden Zielmoleküle 3 zu den Orten des affinitätsbindenden Fängermoleküls 2 als auch zur Beseitigung unerwünschter Bindungsereignisse eingesetzt werden.

Die Meßelektroden 6a, 6b sind über deren Anschlüsse mit Meß- und Auswertevorrichtungen verbindbar. So sind die Meßelektroden 6a, 5b - beispielsweise zu Zwecken der Beaufschlagung mit elektrischen Potentialen bzw. zur Ableitung von elektrischen Potentialen bzw. zur Polarisierung - z. B. mit einem Multipotentiostaten verbunden, wobei eine Polarisation der Meßelektroden 6a, 6b in Bereichen von -1.000 mV bis +1.000 mV, insbesondere -500 mV bis +500 mV, vorzugsweise -300 mV bis +300 mV, ganz besonders bevorzugt -200 mV bis +200 mV, möglich ist. Dabei kann sowohl eine Gleichspannung als auch eine Wechselspannung - je nach Meßverfahren - an die Meßelektroden 6a, 6b angelegt sein. Von diesen Werten kann jedoch deutlich abgewichen werden, sofern anwendungsbezogen erforderlich, wobei die Frequenz der Wechselspannung breit variiert werden kann. Im Rahmen der vorliegenden Erfindung ist es bevorzugt, daß die Detektionseinheit 1 mit einer Vielzahl von - beispielsweise mit zwei, drei, vier, fünf, zehn, sechzehn oder mehr - Meßeinrichtungen 5 bzw. Elektrodenarrays ausgestattet ist, wobei dann die jeweiligen Meßelektroden 6a, 6b bzw. die Elektrodenpaare 7 unabhängig voneinander ansteuerbar und auslesbar sind, beispielsweise mittels des zuvor genannten Multipotentiostaten.

In erfindungsgemäße bevorzugter Weise sind die Meßelektroden 6a, 6b derart angeordnet, daß sie im sogenannten sub-Mikrometer-Bereich (sub-µm-Bereich) voneinander beabstandet sind. Mit anderen Worten sind die Meßelektroden 6a, 6b vorzugsweise weniger als 10 µm, insbesondere weniger als 7 µm, vorzugsweise weniger 5 µm, bevorzugt weniger als 3 µm, besonders bevorzugt weniger als 1 µm, voneinander beabstandet. Im allgemeinen sollten die Meßelektroden 6a, 6b so eng zueinander angeordnet werden, daß sie zumindest im wesentlichen der Größe großer Molekülkomplexe, z. B. Immunoproteinen oder DNA-Molekülen, nahekommen. Dies führt zu dem Vorteil, daß sich zwischen nahe benachbarten Meßelektroden 6a, 6b beispielsweise elektrische Wechselfelder erzeugen lassen und der resultierende Strom hauptsächlich von den zu detektierenden Molekülen bzw. Molekülkomplexen im elektrodennahcn Raum beeinflußt wird. Die Form bzw. Feinstruktur der Meßelektroden 6a, 6b kann erfindungsgemäß in weiten Bereichen variieren, wobei beispielsweise eine interdigitale Ausbildung bzw. Anordnung erfindungsgemäß bevorzugt ist. Gleichermaßen können die Meßelektroden 6a, 6b als parallele Streifen oder mäanderförmige oder runde oder schneckenartige Strukturen bzw. Formen ausgebildet sein. Die Meßelektroden 6a, 6b sind vorzugsweise zum Meßraum hin nicht abgedeckt, während die Zuleitungen zu den Meßelektroden 6a, 6b vorzugsweise elektrisch isoliert sind. Die Breite der Meßelektroden 6a, 6b kann in weiten Bereichen variieren, so kann die Breite der Meßelektroden 6a, 6b beispielsweise 50 bis 1.000 nm, vorzugsweise 100 bis 800 nm, vorzugsweise 150 bis 500 nm, besonders bevorzugt 200 bis 300 nm betragen. Prinzipiell sind aber sowohl größere als auch kleinere Elektrodenbreiten erfindungsgemäß geeignet.

Wie die Figur weiterhin zeigt, kann die Meßeinrichtung 5 als ein Elektrodenarray ausgebildet sein, wobei das Elektrodenarray mindestens zwei Meßelektroden 6a, 6b und gegebenenfalls als weiteren Bestandteil einen Abschnitt des Trägers 4 umfaßt Gemäß einer weiteren erfindungsgemäß bevorzugten Ausführungsform weist die Meßeinrichtung 5 eine Vielzahl derartiger Elektrodenarrays auf. Durch die Strukturierung der Meßeinrichtung 5 in Elektrodenarrays resultiert eine Kompartimentierung, wobei die Randbereiche des Elektrodenarrays, welche beispielsweise durch den Träger 4 gebildet werden können, erhöht sein können. Darüber hinaus ist es aber auch möglich, daß eine Kompartimentierung durch das Aufbringen beispielsweise weiterer Polymerschichten oder von Distanzringen realisiert ist. Sofern anwendungsbezogen erforderlich, kann die Meßeinrichtung 5 bzw. das Elektrodenarray auch eine Vielzahl von Meßelektroden 6a, 6b, d. h. beispielsweise zwei, drei, vier, fünf, sechs oder mehr, aufweisen, wodurch eine gewisse Signalverstärkung bei der Detektion von Zielmolekülen 3 erreicht werden kann. Gemäß einer erfindungsgemäßen Ausführungsform kann die Detektionseinheit 1 beispielsweise 15 Elektrodenarrays aufweisen, von denen jeweils sieben Elektrodenarrays mit identischen Fängermolekül 2 beaufschlagt sind und ein Elektrodenarray als Referenz fungiert, so daß mit einer derartigen Detektionseinheit 1 insgesamt sieben Doppelbestimmungen sowie eine Referenzbestimmung durchführbar ist.

Gemäß einer alternativen erfindungsgemäßen Ausführungsform umfaßt die Meßeinrichtung 5 der erfindungsgemäβen Detektionseinheit 1 mindestens zwei Paare 7 von Meßelektroden 6a, 6b (nicht dargestellt). Bei einer solchen Konfiguration ist es möglich, daß die jeweiligen Paare 7 von Meßelektroden 6a, 6b jeweils mit unterschiedlichen Fängermolekülen 2 beaufschlagt sind, so daß innerhalb einer Meßeinrichtung 5 mehrere Fängermoleküle 2 auf die unterschiedlichen Paaren 7 von Meßelektroden 6a, 6b aufgebracht werden können, so daß eine zu analysierende Probe auf das Vorliegen mehrerer voneinander verschiedener Zielmoleküle 3 bzw. Markermoleküle in einer Meßeinrichtung 5 untersucht werden kann. Dabei sind die Paare 7 von Meßelektroden 6a, 6b jeweils unabhängig voneinander ansteuerbar. Auf diese Weise ist es beispielsweise möglich, daß die Meßeinrichtung 5 zwei, drei, vier oder mehr Paare 7 von Meßelektroden 6a, 6b aufweist, die jeweils mit unterschiedlichen Fängermolekülen 2 beaufschlagt sind, so daß die Meßeinrichtung 5 zwei, drei, vier oder mehr Arten bzw. Spezies von Fängermolekülen 2 umfaßt, so daß eine zu analysierende Probe in bezug auf das Vorhandensein von zwei, drei, vier oder mehr Arten bzw. Spezies von Zielmoleküle 3 untersuchbar ist. Weiterhin ist es erfindungsgemäß möglich, daß die Meßeinrichtung 5 mindestens zwei, jeweils ein aus zwei Meßelektroden 6a, 6b gebildetes Elektrodenpaar 7 aufweisende Elektrodenarrays umfaßt. Bei dieser Ausführungsform sind die Elektrodenarrays vorzugsweise mit jeweils einem Fängermolekül 2 beaufschlagt. Gemäß dieser Anordnung ist es auch möglich, die Elektrodenarrays mit jeweils einem Fängermolekül 2 gemäß der vorgenannten Definition (d. h. als Mischung verschiedener Biokomponenten) auszustatten. Diese Anordnung eignet sich insbesondere zur Analyse mehrerer Proben, da die Detektionseinheit 1 aufgrund des Vorhandenseins der Elektrodenarrays kompartimentiert ist.

Gemäß einer weiteren alternativen Ausführungsform kann die Meßeinrichtung 5 mindestens ein Elektrodenarray, welches mindestens zwei aus jeweils zwei Meßelektroden 6a, 6b gebildete Elektrodenpaare 7 aufweist, umfassen. In diesem Fall können die jeweiligen Elektrodenpaare 7 - wie zuvor geschildert - mit jeweils einem Fängermolekül 2 (d. h. mit einer Art bzw. Spezies von Fängermolekül bzw. Biokomponente) beaufschlagt sein. Auf diese Weise können auf einer Detektionseinheit 1 mehrere unterschiedliche Proben auf das Vorliegen von mehreren, voneinander verschiedenen Zielmolekülen 3 untersucht werden.

Für die zuvor beschriebenen Fälle bzw. Ausgestaltungen sollten die einzelnen Elektrodenpaare 7 unabhängig voneinander mit elektrischen Potentialen beaufschlagbar sein, so daß Meßeffekte an den einzelnen Meßelektroden 6a, 6b der Paare 7 bzw. an den einzelnen Elektrodenpaaren 7 unabhängig voneinander ableitbar sind.

Die Erfassung des vorzugsweise elektrischen Meßsignals bzw. der Meßeffekte, welche bei oder durch Interaktion bzw. Wechselwirkung der Fängermoleküle 2 mit den Zielmolekülen 3 erzeugt wird, erfolgt vorzugsweise potentiometrisch und/oder voltametrisch und/oder konduktometrisch. Darüber hinaus ist auch eine amperometrische und/oder impedimetrische Erfassung des Meßeffektes möglich, beispielsweise mittels Impedanzspektrometrie.

Die ermittelten Meßsignale werden über die Meßeinrichtung 5 abgeleitet, gegebenenfalls verstärkt bzw. gemittelt und vorzugsweise einer mikroprozessorgestützten Auswertung - vorzugsweise unter Verwendung entsprechender Kontroll- bzw. Auswertungssteuerungsprogramme - unterzogen. Die diesbezüglich einsetzbaren Vorrichtungen sind dem Fachmann hinlänglich bekannt, und der Fachmann ist jederzeit in der Lage, die entsprechenden Komponenten in bezug auf die Detektionseinheit 1 abzustimmen. Zur Messung der Meßeffekte können Gleichströme und vorzugsweise Wechselströme mit vorgegebener Frequenz auf die Meßelektroden 6a, 6b bzw. an die Elektrodenpaare 7 appliziert sein, wobei die Frequenz des Wechselstroms 0,1 Hz bis 1 MHz oder mehr betragen kann. In diesem Zusammenhang werden die mit den Fängermolekülen 2 bzw. gegebenenfalls den Fängennolekül/Zielmolekül-Komplexen beaufschlagten Elektrodenpaare 7 bzw. Meßelektroden 6a, 6b beispielsweise mittels Impedanzspektrometrie vermessen, wobei Kapazität, Leitfähigkeit und Dielektrizitätskonstante sowie der Phasenwinkel durch die Messung und die nachfolgende Auswertung ermittelbar sind.

Die Detektionseinheit 1 zeichnet sich dadurch aus, daß die Wechselwirkung von Fängermolekülen 2 einerseits und Zielmolekülen 3 andererseits zur Erzeugung eines insbesondere über die Meßelektroden 6a, 6b ableitbaren elektrischen Meßsignals führt, welches über eine entsprechende Auswerteelektronik erfaßbar und analysierbar ist. Weiterhin ist es möglich, daß ein insbesondere auf die Meßelektrode 6a, 6b beaufschlagtes Potential und/oder ein beaufschlagter Strom durch die Wechselwirkung von Fängermolekülen 2 einerseits und Zielmolekülen 3 andererseits veränderbar ist, beispielsweise durch eine Kapazitäts- und/oder Widerstands- und/oder Impendanzänderung des Systems, wobei diese Änderung meßtechnisch ebenfalls erfaßbar und analysierbar ist.

Gemäß einer erfindungsgemäß bevorzugten Ausführungsform kann es vorgesehen sein, daß das insbesondere elektrische Meßsignal mittels einer infolge der Wechselwirkung von Fängermolekülen 2 einerseits und Zielmolekülen 3 andererseits induzierten chemischen Reaktion, insbesondere Redoxreaktion bzw. elektrochemischen Reaktion, generierbar ist. Bei der chemischen Reaktion kann es sich insbesondere um eine enzymkatalysierte Reaktion handeln, wobei die Zielmoleküle 3 vor oder nach der Wechselwirkung mit den Fängermolekulen 2 beispielsweise mit einem Enzym gekoppelt sind, welches anschließend ein gegebenenfalls zuzugebenes Substrat derart umsetzt, daß auf diese Weise oder aufgrund nachfolgender Reaktionen ein Stromfluß erfolgt. Mit anderen Worten kann das Zielmolekül 3 mit einem Enzym beispielsweise verbunden bzw. "gelabelt" sein, wobei bei Einsatz von Enzymsubstraten die Freisetzung eines elektrochemisch reversiblen Produktes durch das Enzym katalysiert wird. Bei einer solchen Meßanordnung kann z. B. eine Meßelektrode (z. B. Meßelektrode 6a) eines Elektrodenpaars 7 derart polarisiert werden, daß ein reduziertes Reaktionsprodukt oxidiert wird, und die andere Meßelektrode (z. B. Meßelektrode 6b) desselben Elektrodenpaars 7 kann derart polarisiert werden, daß ein oxidiertes Reaktionsprodukt reduziert wird, so daß ein zyklischer Prozeß vorliegt, der gewissermaßen zu einer Verstärkung des Meßsignals führt.

Im Rahmen der Redox-Rezyklierung ("Redox-Recycling") werden vorzugsweise sogenannte Enzymmarker eingesetzt, die - wie zuvor angesprochen - entweder mit den Zielmolekülen 3 an die auf der Meßelektrode 6a, 6b beaufschlagten Fängermoleküle 2 in das Meßsystem eingeführt werden oder nach der erfolgten spezifischen Bindung zwischen Fängermolekülen 2 einerseits und Zielmolekülen 3 andererseits durch sekundäre Bindungsprozesse, wie Antikörperbindung, Interkalation, kovalente Anheftung und andere gebräuchliche Markierungsreaktionen, vorzugsweise an die Zielmoleküle 3 gebunden werden. Das entsprechende Enzym, welches sich somit vorzugsweise nur an solchen Meßeinrichtungen 5 befindet, an denen die molekulare Erkennungsreaktion zwischen Fängermolekülen 2 einerseits und Zielmolekülen 3 andererseits stattgefunden hat, werden anschließend mit einem elektrochemisch inaktiven Substrat, z. B. p-Aminophenylphosphat versetzt, welches dann durch die Enzymreaktion, beispielsweise unter Verwendung einer alkalischen Phosphatase, in ein elektrodenaktives, der Redox-Rezyklierung zugängliches Produkt, beispielsweise p-Aminophenol, umgewandelt wird.

Im Rahmen des zyklischen Redoxprozesses mit einer als Anode polarisierten Meßelektrode (z. B. Meßelektrode 6a) und einer als Kathode polarisierten Meßelektrode (z. B. Meßelektrode 6b) des Elektrodenpaars 7 und induzierter Oxidation bzw. Reduktion des elektrodenaktiven Produkts wird insgesamt ein Summenstrom erhalten, der mit der Menge der an der entsprechenden Elektrodenposition gebundenen Zielmoleküle 3 korreliert. Bezogen auf die gebundenen Zielmoleküle 3, sollte die Menge an eingeführtem Markerenzym vorzugsweise quantitativ und/oder stöchiometrisch sein. Sofern man die zur Immobilisierung hergestellten Mikrokompartimente auch zur Volumentrennung bei dieser Art von Detektion nutzt, ist die in jedem Mikrokompartiment bzw. Elektrodenarray entstehende Konzentration an elektrodenaktiven Substanzen ein quantitatives Maß für die Zahl der an dieser Position individuell stattgefundenen Erkennungsreaktionen. Auf diese Weise bedeutet somit das Fehlen einer elektrochemischen Reaktion auch das Ausbleiben eines Erkennungsereignisses und damit die Abwesenheit bzw. Nichtbindung der Zielmoleküle 3 in bezug auf die entsprechenden Fängermoleküle 2.

Mit Hilfe der sogenannten Redox-Rezyklierung ist gleichermaßen eine gewisse Verstärkung des Meßsignals realisierbar, da einerseits das gebundene Enzym, insbesondere die alkalische Phosphatase, eine große Menge des Substrats in das elektrodensensitive Produkt überführt und dieses Produkt dann in einer Vielzahl von zyklischen Prozessen oxidiert bzw. reduziert wird.

Was die zu analysierende Probe betrifft, mit welcher die Detektionseinheit 1 beaufschlagbar ist, so handelt es sich hierbei um eine Probe aus biologischem bzw. körpereigenem Material, wobei diesbezüglich Körperflüssigkeiten, wie Blut oder Urin, oder Körpergewebe, wie Tumorgewebe, in Betracht kommen. Beispielsweise kann für die Bestimmung des zuvor beschriebenen PSA-Moleküls eine Blutprobe zugrundegelegt werden, während für die Bestimmung tumorspezifischer Markerproteine, wie Angiogenesefaktoren und dergleichen, vorzugsweise eine Probe des Tumors an sich verwendet wird. Zur Beaufschlagung auf die erfindungsgemäß eingesetzte Detektionseinheit 1 kann es erfindungsgemäß vorgesehen sein, daß die Probe aufgearbeitet wird. Im allgemeinen ist es möglich, daß die Probe als Gewebe oder aber in Form von isolierten Zellen, lysierten Zellen, Membranfragmenten, isolierten Proteinen, isolierten Nukleinsäuren und dergleichen eingesetzt wird. Erfmdungsgemäß kann die Beaufschlagung der Detektionseinheit 1 mit der Probe beispielsweise mittels Mikrofluidiksystemen, insbesondere einem Nanoliterdispensierautomaten, vorzugsweise rechnergesteuert, beaufschlagt werden. Bei der Untersuchung bzw. Detektion von DNA bzw. RNA kann eine Amplifikation, insbesondere eine primergestützte lineare Amplifikation, durchgeführt werden. Diese ist aber rein fakultativ; so ist bei einer spezifischen Untersuchung auf 16S-RNA eine derartige Amplifikation in der Regel nicht erforderlich, da 165-RNA in den zu untersuchenden Medien bzw. Bakterien in hohen Kopiezahlen vorliegt. Die Aufarbeitung der Probe ist dem Fachmann an sich bekannt, so daß es diesbezüglich keiner weiteren Ausführungen bedarf.

Was das Material des Trägers 4 der erfindungsgemäß eingesetzten Detektionseinheit 1 betrifft, so handelt es sich hierbei vorzugsweise um ein elektrisch isolierendes und gegenüber den zu untersuchenden Zielmolekülen 3 vorzugsweise inertes Material. Insbesondere handelt es sich bei dem Material des Trägers 4 um eine Siliziumverbindung, Glas, Keramik bzw. organische Polymere. Wie zuvor ausgeführt, ist der Träger 4 hinsichtlich seiner Formgebung kompartimentiert sein, wobei einzelne Kompartiment, welche mit Meßelektroden 6a, 6b bzw. Elektrodenpaaren 7 ausrüstbar sind, als Mikroarrays ausgebildet sind. Demgegenüber ist aber auch eine flache, planare Ausbildung des Trägers 4 möglich. Vorzugsweise dient der Träger 4 zur mechanischen

Unterstützung der Meßelektroden 6a, 6b, Die Verwendung einer Siliziumverbindung ist zudem bevorzugt, wenn beispielsweise zur individuellen Kontrolle der Elektrodenpositionen, beispielsweise in bezug auf das Elektrodenarray, Steuerung und Schaltung sowie Auslesung der einzelnen Meßelektroden 6a, 6b, zusätzliche elektronische Elemente, wie Transistoren, Dioden, Widerstände und andere übliche elektronische Komponenten, positionsbezogen im Träger 4 integriert sind.

Im Rahmen der vorliegenden Erfindung kann als Ausgangsvorrichtung für die erfindungsgemäß eingesetzte Detektionseinheit 1 ein Biosensor verwendet werden, wie er in der WO 94/29708 A1 sowie der zugehörigen US 5 670 031 A und der DE 43 18 519 C2, in der WO 97/34140 A1 sowie der zugehörigen US 2002/28441 A1 und der DE 196 10 115 C2, in der WO 2000/67026 A1 sowie der zugehörigen US 6 881 379 B1 und der DE 199 16 867 A1, in der WO 00/62048 A2 sowie der zugehörigen DE 199 16 921 A1 und schließlich in der DE 196 28 052 C1 beschrieben ist, wobei die in den vorgenannten Druckschriften genannten Biosensoren in der erfindungsgemäßen Art und Weise ausgestattet werden müssen. Im Rahmen der vorliegenden Erfindung können die aus dem Stand der Technik bekannten Biosensoren in erfindungsgemäßer Weise mit den zuvor definierten speziellen Fängermolekülen ausgerüstet bzw. beaufschlagt werden, um für die erfindungsgemäßen Zwecke nutzbar zu sein. Die Ausrüstung der Biosensoren mit den erfindungsgemäß ausgewählten Fängermolekülen bzw. Biokomponenten ist dem Fachmann als solche bekannt, so daß bezüglich herstellungstechnischer Eigenschaften hierauf nicht näher eingegangen zu werden braucht.

Die vorliegende Erfindung - gemäß einem **ersten** Aspekt der vorliegenden Erfindung - betrifft somit ein Verfahren zur Herstellung einer Referenzdatenbank für Krankheiten aus der Gruppe von Tumorerkrankungen, insbesondere Krebserkrankungen, und entzündlichen Erkrankungen des Urogenitaltraktes mit einer Vielzahl von krankheitsspezifischen Profilen einer Vielzahl von Patienten, wobei das erfindungsgemäße Verfahren durch die folgenden Verfahrensschritte gekennzeichnet ist:
a) Untersuchung einer gegebenenfalls aufgearbeiteten Probe, bereitgestellt aus biologischem bzw. körpereigenem Material eines Patienten, insbesondere ausgewählt aus Körperflüssigkeiten, insbesondere Blut bzw. Urin, oder Körpergewebe, insbesondere Tumorgewebe, auf Vorhandensein spezifischer Zielmoleküle, wie zuvor definiert, insbesondere zur Erstellung eines spezifischen Profils von Zielmolekülen (Markerprofil), unter Verwendung einer zuvor definierten Detektionseinheit; dann
b) Korrelation der in Verfahrensschritt a) ermittelten Zielmoleküle, insbesondere des in Verfahrensschritt a) erstellten Profils von Zielmolekülen (Markerprofil), mit dem der Probe zuzuordnenden pathologischen bzw. klinischen bzw. symptomatischen bzw. histologischen Befund des betreffenden Patienten; dann
c) Einstellung der in den Verfahrensschritten a) und/oder b) ermittelten bzw. herangezogenen Daten in die Referenzdatenbank; anschließend
d) Wiederholung der Verfahrensschritte a) bis c) an einer Vielzahl von Patienten; und schließlich
e) gegebenenfalls Einstellung der ermittelten Zielmoleküle, insbesondere des in Verfahrensschritt a) erstellten Profils von Zielmolekülen (Markerprofil), in verschiedene Gruppen, insbesondere verschiedene Gruppen jeweils gleicher oder zumindest ähnlicher Korrelation.

Der Begriff "Korrelation" ist in diesem Zusammenhang so zu verstehen, daß die mittels der Detektionseinheit ermittelten Zielmoleküle bzw. das Profil der Zielmoleküle (Markerprofil) einer Probe (beispielsweise einer Tumorprobe) eines Patienten dem krankheitsspezifischen Profil des entsprechenden Patienten zugeordnet wird. Dies kann beispielsweise und in nichtbeschränkender Weise dergestalt sein, daß beispielsweise anhand statistischer Auswertungen an einer Vielzahl von Patienten einem bestimmten Zielmolekül bzw. Markermolekül ein erhöhtes Rezidivrisiko in bezug auf die Tumorerkrankung zugeordnet wird. Diese aufgrund einer Vielzahl statistischer Daten angenommene Korrelation kann dann als Grundlage für eine Aussage hinsichtlich einer Rezidivabschätzung in bezug auf einen Patienten herangezogen werden, in dessen Probe das entsprechenden Zielmoleküle bzw. Markermolekül nachgewiesen wird.

Mit anderen Worten besteht das Prinzip der erfindungsgemäßen Referenzdatenbank darin, daß zunächst eine möglichst große Anzahl von beispielsweise Tumorgewebsproben auf das Vorhandensein der oben angeführten Zielmoleküle bzw. Markermoleküle bzw. Tumormarker (d. h. das Markerprofil) überprüft wird, um auf diese Weise einen Einfluß der Zielmoleküle auf den Krankheitsverlauf des Patienten zu ermitteln, Die so gewonnenen Zusammenhänge bzw. der entsprechende Nachweis des Einflusses des Zielmoleküls auf den Krankheitsverlauf können dann als Referenz in bezug auf weitere Proben anderer Patienten übertragen bzw. herangezogen werden.

Diesbezüglich kann beispielsweise und in nichtbeschränkender Weise derart verfahren werden, daß beispielsweise Paraffinmaterial von histopathologisch sowie klinisch im Rahmen einer operativen Diagnostik/Therapie und Nachsorge dokumentierten Patienten - beispielsweise mit nachgewiesenem Harnblasenkarzinom - in Form eines sogenannten *Tissue Microarrays* (TMA), welches eine Ausführungsform der erfindungsgemäßen Detektionseinheit darstellt, untersucht wird. Dabei werden nahezu sämtliche derzeit bekannten Tumormarker mit Einfluß auf den individuellen Krankheitsverlauf an einer Vielzahl von Tumorproben getestet. Die darauf basierenden Tumorproben und als Referenz herangezogene Proben normalen Gewebes präsentieren die Hamblasenkarzinomerkrankung in bezug auf die gesamte klinische Breite, z. B. in bezug auf die Rezidiv- und Progressionswahrscheinlichkeit. In diesem Zusammenhang kann die erfindungsgemäß eingesetzte Detektionseinheit bzw. der Tissue Microarray (TMA) aus zwei unterschiedlichen Arrays (beispielsweise Meßeinrichtungen oder Elektrodenarrays) bestehen. Ein Array (Rezidiv-TMA) repräsentiert oberflächliche Harnblasenkarzinome mit dokumentiert aufgetretenem Rezidiv. Der zweite Array (Progressions-TMA) entspricht aggressiven Tumoren mit dokumentierter Tumorprogression oder Tod infolge des Tumors. Nach Analyse der Ergebnisse können die so definierten Antikörper (Fängermoleküle) auf die erfindungsgemäße Detektionseinheit aufgebracht werden, um so die Analyse von Gewebeproben oder Körperflüssigkeiten zu ermöglichen und um auf diese Weise eine individuelle Prognoseabschätzung in bezug auf das Rezidivrisiko, Progressionsrisiko und dergleichen zu ermöglichen.

Insbesondere in Zusammenhang mit der Erstellung der Referenzdatenbank kann die Probeentnahme bei einem Patienten in bezug auf Tumorgewebe wie folgt durchgeführt werden:
A) Einverständniserklärung über die wissenschaftliche Nutzung von entnommenem Gewebe und der Krankheitsgeschichte durch den Patienten;
B) Probenasservierung im Operationssaal durch Personal der Referenzdatenbank:
   - Präparation eines repräsentativen Tumorgewebestücks und Präparation von Normalgewebe durch geschultes Personal nach Anweisungen des Operateurs
C) Unterleitung des Tumor- und Normalgewebes in drei gleiche Stücke mit Asservierung wie folgt:
   1. Vorbehandlung des Gewebes, beispielsweise mit Histo-Frees-Spray, Einbringen des ersten Gewebepräparates in Flüssigstickstoff (Warmischämiezeit < 3 Minuten); es resultiert eine erste Gewebetube,
   2. Einbringen des zweiten Gewebestücks in Formalin und Weitergabe an die Pathologie für eine Referenzhistologie und Aufbewahrung bei Raumtemperatur; es resultiert eine zweite Gewebetube,
   3. Einbringen des dritten Gewebepräparates in RNAlater und Aufbewahrung bei Kühlschranktemperatur für 24 Stunden und anschließend bei -25 °C; es resultiert eine dritte Gewebetube;
   4. Zusätzlich werden von dem jeweiligen Patienten eine Monovette-EDTA Blut und eine Serummonovette abgenommen (je nach Tumorentität wird zusätzlich eine Urinprobe genommen) und Aufbewahrung unter Tiefkühlung bei - 20 °C;
   5. Erstellung eines Einfrierprotokolls
D) Probenasservierung und Verwaltung in der Referenzdatenbank:
   1. Aufbewahrung des Gewebes in der zentralen Referenzdatenbank bzw. Tumordatenbank;
   2. Erstellung eines Markerprofils in zuvor beschriebener Weise;
   3. Verwaltung des Gewebes und der Patientendaten gemäß einem definierten, insbesondere EDV-gestützten Muster;
   4. Bereitstellung des Gewebes zur Aufbereitung.

Auf diese Weise wird eine standardisierte Asservierung des operativ entfernten Gewebes gewährleistet, und die intraoperativ gewonnenen Erkenntnisse bezüglich des bestimmten Marker- bzw. Antigenprofils bleiben für Referenzuntersuchungen durch herkömmliche Verfahren, wie immunohistochemische Färbungen, reproduzierbar.

Weiterhin betrifft die vorliegende Erfindung - gemäß einem **zweiten** Aspekt der vorliegenden Erfindung - ein Verfahren zur Ermittlung des Risikos, an einer Krankheit zu erkranken, bzw. zur Prognose eines Krankheitsverlaufs bzw. zur Prognose von individuellen Arzneimittelwirkungen bei Behandlung einer Krankheit. Bei der der Krankheit handelt es sich - wie zuvor beschrieben um Tumorerkrankungen, insbesondere Krebserkrankungen, des Urogenitaltraktes oder entzündliche Erkrankungen des Urogenitaltraktes. Das erfindungsgemäße Verfahren ist dabei durch die folgenden Verfahrensschritte gekennzeichnet:
a) Zunächst Untersuchung einer gegebenenfalls aufgearbeiteten Probe, bereitgestellt aus biologischem bzw. körpereigenem Material eines Patienten, wobei die Probe eine Körperflüssigkeit, insbesondere Blut bzw. Urin, oder Körpergewebe, insbesondere Tumorgewebe, darstellt, auf Vorhandensein spezifischer Zielmoleküle, wie zuvor definiert, insbesondere Erstellung eines spezifischen Profils von Zielmolekülen (Markerprofil) unter Verwendung einer zuvor definierten Detektionseinheit; anschließend
b) Auswertung, insbesondere Abgleich der spezifischen Zielmoleküle bzw.
   des Profils von Zielmolekülen (Markerprofil) mit der zuvor beschriebenen Referenzdatenbank (wobei die Referenzdatenbank entsprechende Markerprofile einer Vielzahl von Patienten und das hiermit korrelierende Krankheitsbild, insbesondere den dem Markerprofil zuzuordnenden pathologischen und/oder klinischen symptomatischen und/oder histologischen Befund, erfaßt bzw. enthält); schließlich
c) Erstellung einer Diagnose der Krankheit bzw. Ermittlung eines Risikos, an einer Krankheit zu erkranken, bzw. Prognose eines Krankheitsverlaufs bzw. Prognose von individuellen Arzneimittelwirkungen bei Behandlung einer Krankheit auf Grundlage der Auswertung bzw. Abgleich der spezifischen Zielmoleküle bzw. des Profils von Zielmolekülen (Markerprofil) mit der Referenzdatenbank.

Für weitere Ausführungen betreffend das erfindungsgemäße Verfahren gemäß dem zweiten Aspekt der vorliegenden Erfindung kann auf obige Ausführungen betreffend die erfindungsgemäß eingesetzte Detektionseinheit sowie das erfindungsgemäße Verfahren gemäß dem ersten Aspekt der vorliegenden Erfindung verwiesen werden, welche hier entsprechend gelten.

Die erfindungsgemäß eingesetzte Detektionseinheit sowie die erfindungsgemäße Verfahren weisen zahlreiche Vorteile auf, von denen rein beispielhaft die folgenden genannt werden sollen.
- Bei der erfindungsgemäß eingesetzten Detektionseinheit handelt es sich um einen insbesondere elektrischen Biochip, der individuell mit spezifischen Fängermolekülen zur Detektion spezifischer Zielmoleküle eingesetzt wird. Durch die variable und anwendungsbezogene Ausstattung mit Fängermolekülen kann der Biochip für unterschiedlichste Anwendungen individuell abgestimmt werden.
- Aufgrund des vorzugsweise elektrischen Meßprinzips entfallen weitere Erfassung- bzw. Detektionsvorrichtungen, wie optische Sensoren. Denn die erfindungsgemäß eingesetzte Detektionseinheit vereint in einer Vorrichtung sowohl Erfassungskomponenten für die Zielmoleküle als auch Ausgabekomponenten für das Meßsignals, so daß weitere diesbezügliche Vorrichtungen, wie optische Auswertevorrichtung, entfallen. Hierdurch vereinfacht sich der Aufbau der gesamten Vorrichtung deutlich.
- Aufgrund der kompakten Struktur der erfindungsgemäß eingesetzten Detektionseinheit und der geringen Anzahl an zusätzlichen Vorrichtungen ist der Gesamtaufbau gleichermaßen kompakt, so daß insgesamt ein portables System resultiert, welches vor Ort - beispielsweise in einem Operationssaal - eingesetzt werden kann.
- Mit Hilfe der erfindungsgemäß eingesetzten Detektionseinheit ist eine rasche Identifizierung (Testdauer ca. 20 Minuten) beispielsweise gewebetypischer Marker bei einer Krebsoperation möglich, so daß bereits vor Ort während einer Operation sicher und schnell diese Marker quantifiziert werden können.
- Mit Hilfe des erfindungsgemäß eingesetzten Meßverfahrens zur Bestimmung eines Antigenprofils können bereits beispielsweise intraoperativ Informationen zum Antigenprofil des vorliegenden Gewebes z. B. in Ergänzung zum Ergebnis der herkömmlichen, routinemäßigen Schnellschnittuntersuchung erhalten werden, so daß therapeutische Entscheidungen schon während der Operation getroffen bzw. optimiert werden können. Dies führt zu erheblichen Vorteilen für den zu behandelnden Patienten.
- Insgesamt resultiert somit ein System zur Bestimmung von Markerprofilen, welches eine einfache Bedienung aufweist, gut zu desinfizieren ist und selbstständige Messungen mehrerer paralleler Proben ermöglicht. Zudem ist eine Anbindung eines separaten Miniautomatens zur parallelen Probenvorbehandlung (z. B. käufliches Portalrobotersystem der Fa. Gilson) möglich.

Die vorliegende Erfindung wird anhand des nachfolgenden Ausführungsbeispiels veranschaulicht, welches die vorliegende Erfindung jedoch keinesfalls beschränken soll.

### AUSFÜHRUNGSBEISPIEL:

### Ausführungsbeispiel der Bespottung der Siliziumchips am Beispiel VEGF:

| | | |
|---|---|---|
| Geräte: | 1. | Stereo-/Auflichtmikroskop (z. B. Nikon SMZ 1000) |
| | 2. | Bespottungseinrichtung bestehend aus Mikromanipulator mit Injektionseinheit (z. B. eppendorf FemtoJet + eppendorf InjectMan NI2) |
| | 3. | eBiochip Analysegerät Fraunhofer Institut |
| | 4. | Computersoftware Origin und MCDDE32 zum Betrieb des Analysegerätes |
| | 5. | Gewebehomogenisator zur Tumorgewebeaufarbeitung (z. B. Quiagen TissueLyser) |
| Lösungen: | 1. | Antikörperlösung (z. B. VEGF) |
| | 2. | Sulfo-NHS-LC-Biotin zur Biotinylierung der Antikörperlösung |
| | 3. | TBS Puffer (pH 7 und pH 8) |
| | 4. | TTBS Puffer (pH 7) |
| | 5. | TTBS-BSA Puffer (1%ig) (pH 7) |
| | 6. | Glycin/HCl (pH 2) |
| | 7, | pAPP Substrat |
| | 8. | Anti-SEB-Biotin 1:250 |
| | 9. | Extravidin-AP-Konjugat 1:500 |

### Ablauf der Bespottung:

Die Kapillarnadel der Injektionseinheit wird mit der Antikörperlösung (pAK 1:1 1 in PBS ca. 1 mg/ml) befüllt und in den InjectMan eingespannt. Die vorgefertigten Siliziumchips werden unter dem Auflichtmikroskop fixiert und eingestellt. Anschließend erfolgt die Applikation der Antikörperlösung unter manueller Einstellung mittels des Mikromanipulators mit einer Konzentration von ca. 1 mg/ml druck- und zeitgesteuert, so daß sich eine Menge von 30 nl Antikörperlösung pro Meßposition ergibt. Die Positivkontrollposition wird mit a-fd bacteriophage-Biotin 1:250 in PBS bespottet. Die Negativkontrollposition werden mit 1 % BSA in PBS bespottet. Nach Bespottung der 16 Meßpositionen wird der Chip mit PBS abgedeckt, so daß eine Lagerung über einige Stunden möglich ist.

### Ablauf der Gewebeaufarbeitung:

Zur Gewebeaufarbeitung wird ein etwa 5 x 5 mm große Tumorgewebsstück zusammen mit drei 3 mm-Edelstahlbeats in ein Rundboden-Eppendorf-Cup gegeben und mit einer Frequenz von 30/Min über 600 Sekunden durch Schwingungen zerkleinert (Quiagen TissueLyser). Der Überstand wird abpippetiert und dient als Probe für die Proteinmessung.

### Ablauf der Messung:

Die Messung erfolgt vollautomatisch innerhalb des eBiochip Analysegerätes mithilfe der oben genannten Analysesoftware. Hierzu wird der bespottete Siliziumchip in das Anlaysegerät eingelegt und die einzelnen Lösungen, ähnlich dem Verfahren bei einem Sandwich-ELISA, in definierter Folge und Menge über die Meßpunkte des Siliziumchips gegeben. Die Ergebnisse werden durch die Analysesoftware graphisch in Form von Balkendiagrammen dargestellt, die die Antigen/Antikörperreaktion und -bindung auf jeder der 16 Meßpositionen sichtbar machen.

## Patentansprüche

1. Verfahren zur Erstellung einer Referenzdatenbank für Krankheiten aus der Gruppe von Tumorerkrankungen des Urogenitaltraktes mit einer Vielzahl von krankheitsspezifischen Profilen einer Vielzahl von Patienten, **gekennzeichnet durch** die folgenden Verfahrensschritte:
a) Untersuchung einer gegebenenfalls aufgearbeiteten Probe, bereitgestellt aus biologischem bzw. körpereigenem Material eines Patienten, insbesondere ausgewählt aus der Gruppe von Körperflüssigkeiten, insbesondere Blut und/oder Urin, oder Körpergewebe, insbesondere Tumorgewebe, auf Vorhandensein spezifischer Zielmoleküle (3), insbesondere Erstellung eines spezifischen Profils von Zielmolekülen (3) (Markerprofil), unter Verwendung einer Detektionseinheit (1);
b) Korrelation der in Verfahrensschritt a) ermittelten Zielmoleküle (3), insbesondere des in Verfahrensschritt a) erstellten Profils von Zielmolekülen (3) (Markerprofil), mit dem der Probe zuzuordnenden pathologischen und/oder klinischen und/oder symptomatischen und/oder histologischen Befund des betreffenden Patienten;
c) Einstellung der in den Verfahrensschritten a) und/oder b) ermittelten bzw. herangezogenen Daten in die Referenzdatenbank;
d) Wiederholung der Verfahrensschritte a) bis c) an einer Vielzahl von Patienten;
e) gegebenenfalls Einteilung der ermittelten Zielmoleküle (3), insbesondere des in Verfahrensschritt a) erstellten Profils von Zielmolekülen (3) (Markerprofil), in verschiedene Gruppen, insbesondere verschiedene Gruppen jeweils gleicher oder zumindest ähnlicher Korrelation;
wobei:
- die Zielmoleküle (3) für das Auftreten und/oder Vorliegen eines Urothelkarzinoms, Prostatakarzinoms und/oder Nierenkarzinoms charakteristisch sind, wobei die Zielmoleküle (3) Antigene sind, ausgewählt aus der Gruppe von: PSA; E-Cadherin; Catenin; Wachstumsfaktor in Form von HER-2/neu; Proto-Onkogen in Form von Cyclin-D; Angiogenesefaktoren in Form von VEGF-C, VEGF-D und VEGF-3; Wachstumsfaktor in Form von EGF-R; Tumorsuppressorgenprodukten in Form von p53 und PTEN; Zellzyklusregulatoren in Form von p27Kip und K167; Chemokinrezeptoren in Form von CXCR4 und CCR7; Signaltransduktionsfaktoren in Form von Rho-Kinase, Rho A, Rho B, Rho C, Epac 1, Epac 2, H-Ras, Raf-Kinase, Rap 1 und Rap 2; Zellmotilitätsfaktoren in Form von Pecam und Vimentin; Angiogenesefaktor in Form von UPA (PLAU); sowie deren Mischungen und/oder Kombinationen, und
- wobei die Detektionseinheit (1) ausgebildet ist als eine Detektionseinheit (1), insbesondere Biosensor, zur Erzeugung eines elektrischen Meßsignals infolge einer Wechselwirkung zwischen Fängermolekülen (2) einerseits und den Zielmolekülen (3) andererseits,
wobei die Detektionseinheit (1) einen Träger (4) aufweist, wobei auf dem Träger (4) eine Meßeinrichtung (5), welche zur Erfassung der Wechselwirkung zwischen den Fängermolekülen (2) einerseits und den Zielmolekülen (3) andererseits sowie zur Erzeugung eines elektrischen Meßsignals dient, angeordnet ist, wobei die Meßeinrichtung (5) mindestens zwei Meßelektroden (6a, 6b) aufweist, wobei das Material der Meßelektroden (6a, 6b) ausgewählt ist aus der Gruppe von Edelmetallen,
wobei auf der Meßeinrichtung (5) die Fängermoleküle (2) immobilisiert sind, wobei die Fängermoleküle (2) geeignet sind, in spezifischer Weise mit den Zielmolekülen (3) in Wechselwirkung zu treten, wobei die Fängermoleküle (2) Antikörper sind,
wobei die Detektionseinheit (1) mehrere unterschiedliche und getrennt voneinander steuerbare Meßeinrichtungen (5) umfaßt, auf welchen jeweils unterschiedliche Fängermoleküle (2) immobilisiert sind, und wobei die einzelnen Meßeinrichtungen (5) **durch** Kompartimentierungen voneinander getrennt sind.

2. Verfahren nach Anspruch 1, **dadurch gekennzeichnet, daß** die Fängermoleküle (2) auf der Meßeinrichtung (5), vorzugsweise auf den Meßelektroden (6a, 6b) der Meßeinrichtung (5), physikalisch und/oder chemisch, insbesondere chemisch, vorzugsweise mittels Selbstanordnung (Self-Assembling), Elektropolymerisation oder über Gold/Thiol-Bindungen, gebunden sind.

3. Verfahren nach Anspruch 1 oder 2, **dadurch gekennzeichnet, daß** das Material der Meßelektroden (6a, 6b) ausgewählt ist aus der Gruppe von Gold, Platin und/oder Iridium und/oder daß die Meßeinrichtung (5) als ein Elektrodenarray, welches mindestens zwei Meßelektroden (6a, 6b) und gegebenenfalls als weiteren Bestandteil einen Abschnitt des Trägers (4) umfaßt, oder als eine Vielzahl derartiger Elektrodenarrays ausgebildet ist.

4. Verfahren zur Ermittlung eines Risikos, an einer Krankheit zu erkranken, und/oder zur Prognose eines Krankheitsverlaufs und/oder zur Prognose von individuellen Arzneimittelwirkungen bei Behandlung einer Krankheit, wobei die Krankheit ausgewählt ist aus der Gruppe von Tumorerkrankungen des Urogenitaltraktes, **gekennzeichnet durch** die folgenden Verfahrensschritte:
a) Untersuchung einer gegebenenfalls aufgearbeiteten Probe, bereitgestellt aus biologischem bzw. körpereigenem Material eines Patienten, insbesondere ausgewählt aus der Gruppe von Körperflüssigkeiten, insbesondere Blut und/oder Urin, oder Körpergewebe, insbesondere Tumorgewebe, auf Vorhandensein spezifischer Zielmoleküle (3), wie in Anspruch 1 definiert, zur Erstellung eines spezifischen Profils von Zielmolekülen (3) (Markerprofil) unter Verwendung einer Detektionseinheit (1), wie in einem der Ansprüche 1 bis 3 definiert;
b) Auswertung, insbesondere unter Abgleich der spezifischen Zielmoleküle (3), insbesondere des Profils von Zielmolekülen (3) (Markerprofil), mit einer Referenzdatenbank, erstellt gemäß einem Verfahren nach einem der Ansprüche 1 bis 3;
c) Erstellung einer Diagnose der Krankheit und/oder Ermittlung eines Risikos, an einer Krankheit zu erkranken, und/oder Prognose eines Krankheitsverlaufs und/oder Prognose von individuellen Arzneimittelwirkungen bei Behandlung einer Krankheit auf Grundlage der Auswertung und/oder Abgleichung der spezifischen Zielmoleküle (3), insbesondere des Profils von Zielmolekülen (3) (Markerprofil), mit der Referenzdatenbank.

## Claims

1. A method for establishing a reference database for diseases from a group of tumor illnesses of the urogenital tract, having a plurality of disease specific profiles of a plurality of patients, **characterized by** the following process steps:
a) examination of a possibly processed sample, provided from biological material, or material naturally produced in a patient's body, in particular selected from the group of body fluids, in particular blood and/or urine, or body tissue, in particular tumor tissue, for the presence of specific target molecules (3), in particular establishing a specific profile of target molecules (3) (marker profile) using a detection unit (1);
b) correlation of the target molecules (3) identified in process step a), in particular the profile of target molecules (3) (marker profile) established in process step a), with the pathological and/or clinical and/or symptomatic and/or histological findings of the respective patient attributed to the sample;
c) entry of the data identified or utilized in the process steps a) and/or b) into the reference database;
d) repeating process steps a) to c) with a plurality of patients;
e) possibly allocation of the identified target molecules (3), in particular of the profile of target molecules (3) (marker profile) established in process step a) in various groups, in particular various groups of equal or at least similar correlation each;
wherein:
- the target molecules (3) are characteristic for the occurrence and/or presence of a urothelium carcinoma, prostate carcinoma, and/or kidney carcinoma, wherein the target molecules (3) are antigens, selected from the group of: PSA; E-cadherin; catenin; growth factor in the form of HER-2/new; proto-oncogene in the form of cyclin-D; angiogenesis factors in the form of VEGF-C, VEGF-D, and VEGF-3; growth factor in the form of EGF-R; tumor suppressor gene products in the form of p53 and PTEN; cell cycle regulators in the form of p27Kip and Ki67; chemokine receptors in the form of CXCR4 and CCR7; signal transduction factors in the form of Rho kinases, Rho A, Rho B, Rho C, Epac 1, Epac 2, H-Ras, Raf kinases, Rap 1, and Rap 2; cell motility factors in the form of Pecam and Vimentin; angiogenesis factor in the form of UPA (PLAU); as well as the mixtures and/or combinations thereof, and
- wherein the detection unit (1) is embodied as a detection unit (1), in particular a biosensor, for generating an electric measurement signal due to an interaction between radical molecules (2) on one hand, and the target molecules (3) on the other hand,
wherein the detection unit (1) has a carrier (4), wherein a measurement unit (5) is disposed on the carrier, which serves for detecting the interaction between the radical molecules (2) on one hand, and the target molecules (3) on the other hand, as well as for generating an electric measurement signal, wherein the measurement unit (5) has at least two measuring electrodes (6a, 6b), wherein the material of the measurement electrodes (6a, 6b) is selected from the group of precious metals,
wherein the radical molecules (2) are immobilized on the measurement unit (5), wherein the radical molecules (2) are suitable to enter into an interaction with the target molecules (3) in a specific manner, wherein the radical molecules (2) are antibodies,
wherein the detection unit (1) comprises multiple different measurement units (5) that can be controlled separately from each other, on which various radical molecules (2) are each immobilized, and wherein the individual measurement units (5) are separated from each other by means of compartmentalizations.

2. The method according to claim 1, **characterized in that** the radical molecules (2) are physically and/or chemically, in particular chemically bound on the measurement unit (5), preferably on the measurement electrodes (6a, 6b) of the measurement unit (5), preferably by mains of self-assembling, electropolymerization, or via gold/thiol bonds.

3. The method according to claims 1 or 2, **characterized in that** the material of the measurement electrodes (6a, 6b) is selected from the group of gold, platinum, and/or iridium, and/or that the measurement unit (5) is embodied as an electrode array, comprising at least two measurement electrodes (6a, 6b), and possibly comprising a section of the carrier (4) as an integral part, or as a plurality of such electrode arrays.

4. A method for identifying a risk of contracting a disease, and/or for the prognosis of a course of a disease, and/or for the prognosis of the effects of individual pharmaceuticals in the treatment of a disease, wherein the disease is selected from the group of tumor diseases of the urogenital tract, **characterized by** the following process steps:
a) examination of a possibly processed sample, provided from biological material, or material naturally produced in a patient's body, in particular selected from the group of body fluids, in particular blood and/or urine, or body tissue, in particular tumor tissue, for the presence of specific target molecules (3) as defined in claim 1, for establishing a specific profile of target molecules (3) (marker profile) using a detection unit (1), as defined in claims 1 to 3;
b) analysis, in particular in alignment with the specific target molecules (3), in particular of the profile of target molecules (3) (marker profile), with a reference database, compiled in accordance with a method according to one of the claims 1 to 3;
c) compilation of a diagnosis of the disease, and/or assessment of a risk of contracting a disease, and/or prognosis of a course of a disease, and/or prognosis of the effects of individual pharmaceuticals, and/or alignment of the specific target molecules (3), in particular the profile of target molecules (3) (marker profile), with the reference database.

## Revendications

1. Procédé d'établissement d'une base de données de référence pour des maladies du groupe des affections tumorales du tractus urogénital avec une multitude de profiles spécifiques de maladie d'une multitude de patients, **caractérisé par** les étapes de procédé suivantes:
a) examen d'un échantillon, traité le cas échéant, préparé à partir de matériel biologique, respectivement corporel, d'un patient, choisi en particulier parmi le groupe des liquides corporels, en particulier sang et/ou urine, ou des tissus corporels, en particulier de tissus tumoraux, quant à la présence de molécules cibles spécifiques (3), en particulier établissement d'un profil spécifique de molécules cibles (3) (profil de marqueur), moyennant l'utilisation d'une unité de détection (1);
b) corrélation des molécules cibles (3) déterminées dans l'étape a) du procédé, en particulier du profil de molécules cibles (3) établi dans l'étape a) du procédé (profil de marqueur), avec le constat pathologique et/ou clinique et/ou symptomatique et/ou histologique du patient concerné, à affecter à l'échantillon:
c) inscription des données déterminées ou utilisées dans les étapes de procédé a) et/ou b) dans la base de données de référence;
d) répétition des étapes de procédé a) à c) sur une multitude de patients;
e) le cas échéant, répartition des molécules cibles (3) déterminées, en particulier du profil de molécules cibles (3) (profil de marqueur) établi dans l'étape a) du procédé, en différents groupes, en particulier en différents groupes de même corrélation ou au moins de corrélation similaire;
sachant que:
- les molécules cibles (3) sont caractéristiques pour la survenue et/ou la présence d'un carcinome de l'urothélium, d'un carcinome de la prostate et/ou d'un carcinome des reins, les molécules cibles (3) étant des antigènes choisis parmi le groupe de: PSA; E-cadhérine; caténine; facteur de croissance en forme de HER2/neu; proto-oncogène sous la forme de cycline-D; facteurs angiogénétiques sous la forme de VEGF-C, VEGF-D et VEGF-3; facteurs de croissance sous la forme de EGF-R; produits génétiques suppresseurs de tumeurs sous la forme de p53 et PTEN; régulateurs de cycle de cellule sous la forme de p27Kip et Ki67; récepteurs de chimiokine sous la forme de CXCR4 et CCR7; facteurs de transduction de signal sous la forme de Rho-kinase, Rho A, Rho B, Rho C, Epac 1, Epac 2, H-Ras, Raf-kinase, Rap 1 et Rap 2; facteurs de motilité de cellules sous la forme de PECAM et vimentine; facteur d'angiogenèse sous la forme de UPA (PLAU); ainsi que leurs mélanges et/ou leurs combinaisons, et
- sachant que l'unité de détection (1) est configurée comme unité de détection (1), en particulier biocapteur, pour engendrer un signal de mesure électrique à la suite d'une interaction entre des molécules captrices (2) d'une part et des molécules cibles (3) d'autre part,
l'unité de détection (1) comportant un support (4), un dispositif de mesure (5) étant disposé sur le support, qui sert à la saisie des interactions entre les molécules captrices (2) d'une part et les molécules cibles (3) d'autre part, ainsi qu'à engendrer un signal de mesure électrique, le dispositif de mesure (5) comprenant au moins deux électrodes de mesure (6a, 6b), le matériau des électrodes de mesure (6a, 6b) étant choisi parmi le groupe des métaux précieux,
les molécules captrices (2) sur le dispositif de mesure (5) étant immobilisées, les molécules captrices (2) étant appropriées pour entrer de façon spécifique en interaction avec le molécules cibles (3), sachant que les molécules captrices (2) sont des anticorps,
l'unité de détection (1) comprenant plusieurs dispositifs de mesure (5) différents et pouvant être commandés séparément les uns des autres, sur lesquels respectivement différentes molécules captrices (2) sont immobilisées, sachant que les différents dispositif de mesure (5) sont séparés les uns des autres par compartimentage.

2. Procédé selon la revendication 1, **caractérisé en ce que** les molécules captrices (2) sont fixées physiquement et/ou chimiquement, en particulier chimiquement, sur le dispositif de mesure (5), de préférence sur les électrodes de mesure (6a, 6b) du dispositif de mesure (5), de préférence par auto-arrangement (self-assembling), par électropolymérisation, ou par des liaison or/thiol.

3. Procédé selon la revendication 1 ou 2, **caractérisé en ce que** le matériau des électrodes de mesure (6a, 6b) est choisi parmi le groupe de l'or, du platine et/ou de l'iridium et/ou **en ce que** le dispositif de mesure (5) est configuré comme tableau d'électrodes, lequel comprend au moins deux électrodes de mesure (6a, 6b) et, le cas échéant comme autre composant, un tronçon du support (4), ou est configuré comme une multitude de tels tableaux d'électrodes.

4. Procédé de détermination d'un risque de contracter une maladie et/ou de pronostic de l'évolution d'une maladie et/ou de pronostic des effets individuels de médicaments lors du traitement d'une maladie, la maladie étant choisie parmi le groupe des affections tumorales du tractus urogénital, **caractérisé par** les étapes de procédé suivantes:
a) examen d'un échantillon, traité le cas échéant, préparée à partir de matériel biologique, respectivement corporel d'un patient, choisi en particulier parmi le groupe des liquides corporels, en particulier sang et/ou urine, ou des tissus corporels, en particulier de tissus tumoraux, quant à la présence de molécules cibles spécifiques (3), comme défini dans la revendication 1, pour l'établissement d'un profil spécifique de molécules cibles (3) (profil de marqueur), moyennant l'utilisation d'une unité de détection (1), comme défini dans l'une quelconque des revendications 1 à 3;
b) évaluation, en particulier moyennant l'alignement des molécules cibles (3) spécifiques, en particulier du profil de molécules cibles (3) (profil de marqueur), avec une base de données de référence, établie selon un procédé d'après l'une quelconque des revendications 1 à 3;
c) établissement d'un diagnostic de la maladie et/ou détermination d'un risque de contracter une maladie, et/ou pronostic d'une évolution de la maladie et/ou pronostic d'effets individuels de médicaments lors du traitement d'une maladie, sur la base de l'évaluation et/ou de l'alignement des molécules cibles (3) spécifiques, en particulier du profil de molécules cibles (3) (profil de marqueur), avec la base de données de référence.
